# FASCICULE DE BREVET EUROPEEN

(11) **EP 2 297 249 B1**
(45) Date de publication et mention de la délivrance du brevet: **29.11.2017**
(21) Numéro de dépôt: 09746016.6
(22) Date de dépôt: 29.04.2009
(51) Int. Cl.: C09B 61/00, A61K 8/97, A61Q 1/02, A61Q 3/02, A61Q 5/10, A61Q 19/08

(54) **MATIERES COLORANTES D'ORIGINE VEGETALE ET LEUR UTILISATION POUR COLORER DES COMPOSITIONS, EN PARTICULIER COSMETIQUES**
FARBSTOFFE PFLANZLICHEN URSPRUNGS UND IHRE VERWENDUNG FÜR FARBGEBENDE ZUSAMMENSETZUNGEN, INSBESONDERE KOSMETISCHE ZUSAMMENSETZUNGEN
DYESTUFFS OF PLANT ORIGIN AND USE THEREOF FOR COLOURING COMPOSITIONS, IN PARTICULAR COSMETIC COMPOSITIONS

(30) Priorité: 29.04.2008 FR 0852905
(43) Date de publication de la demande: 23.03.2011
(73) Titulaire: LvmH Recherche, 45800 Saint-Jean De Braye (FR)
(72) Inventeur: ANDRE, Patrice, F-45170 Neuville Aux Bois (FR); GARCIA, Michel, F-84360 Lauris (FR)
(74) Mandataire: Chantraine, Sylvie Hélène
(86) Numéro de dépôt international: PCT/FR2009/050794
(87) Numéro de publication internationale: WO 2009/138697

(56) Documents cités:
- EP-A1- 1 191 071
- PIRONE CARY: "Aril Structure and Pigments in the Strelitziaceae (Abstract ID: 564)" BOTANY 2006, [Online] 28 juillet 2006 (2006-07-28), - 2 septembre 2006 (2006-09-02) XP002516321 California State University - Chico Extrait de l'Internet: URL:http://www.2006.botanyconference.org/e ngine/search/index.php?func=detail&aid=564 > [extrait le 2009-02-13] cité dans la demande
- PIRONE CARY: "Two Unique Aril Pigments in the Strelitziaceae (order: Zingiberales). (Abstract ID: 2108)" BOTANY & PLANT BIOLOGY 2007, [Online] 7 juillet 2007 (2007-07-07), - 11 juillet 2007 (2007-07-11) XP002516320 Chicago, Illinois Extrait de l'Internet: URL:http://www.2007.botanyconference.org/e ngine/search/index.php?func=detail&aid=210 8> [extrait le 2007-02-13] cité dans la demande
- DATABASE WPI Week 200741 Thomson Scientific, London, GB; AN 2007-427923 XP002516327 -& JP 2007 112931 A (TOYO INK MFG CO LTD) 10 mai 2007 (2007-05-10) cité dans la demande
- DATABASE WPI Week 2008 Thomson Scientific, London, GB; AN 2008-M13793 XP002516324 LIANFU ZHANG: "method for fast extracting lycopene" -& CN 101 121 631 A (UNIV JIANGNAN [CN]) 13 février 2008 (2008-02-13) cité dans la demande
- DATABASE WPI Thomson Scientific, London, GB; AN 2003-857780 XP002516322 -& JP 2003 277641 A (ICHIMARU PHARCOS INC) 2 octobre 2003 (2003-10-02) cité dans la demande
- PIRONE C ET AL: "Aril Structure and Pigments in the Strelitziaceae (power point presentation)", BOTANY 2006 CALIFORNIA STATE UNIVERSITY - CHICO JULY 28-AUGUST 2, 2006,, 31 July 2006 (2006-07-31), page 21pp, XP009113252,

## Description

La présente invention décrit des nouvelles matières colorantes d'origine végétale, ainsi que leur utilisation pour colorer des compositions, en particulier des compositions cosmétiques.

L'invention s'applique tout particulièrement à la préparation de compositions cosmétiques colorées, en particulier destinées au maquillage de la peau ou des phanères. Elle s'applique également à toute sorte de compositions colorées pour d'autres domaines de l'industrie, tels que le domaine des produits alimentaires, celui des médicaments, des encres, des teintures, des peintures et des produits applicables dans le domaine des arts graphiques et de la décoration en général.

### ETAT DE LA TECHNIQUE

On désigne généralement par le terme « pigments », des substances colorantes, insolubles dans le milieu qu'elles colorent.

D'une façon générale, dans le domaine de la cosmétique, on utilise deux types de pigments.

Plus précisément, on utilise dans le domaine de la cosmétique, et tout particulièrement pour le maquillage, des pigments, notamment d'origine végétale, animale, ou minérale, qui provoquent un effet de coloration par absorption sélective de certaines longueurs d'ondes de la lumière incidente lorsqu'ils sont dispersés dans un milieu, par exemple une composition cosmétique.

Parmi les pigments d'origine végétale, on citera l'indigo, substance colorante extractible obtenue par fermentation de feuilles d'Indigofera suffruticosa ou Indigofera tinctoria.

A noter que l'indigo est pratiquement insoluble dans l'eau et dans l'alcool.

On mentionnera également des composés de type anthocyanes, éventuellement modifiés par des cations métalliques.

L'effet de coloration induit par ces pigments dispersés dans un milieu est provoqué par un phénomène d'absorption sélective de certaines longueurs d'ondes de la lumière incidente par ces composés.

La lumière complémentaire à la lumière absorbée est diffusée par le matériau et en détermine la couleur.

La couleur ainsi produite est souvent appelée « couleur pigmentaire ».

Ces pigments sont largement utilisés pour des préparations dans de nombreux domaines de l'industrie, par exemple dans le domaine des encres ou des peintures, mais aussi dans l'industrie cosmétique, notamment pour le maquillage.

L'article de Cary Pirone intitulé « Aril Structure and pigments in the Strelitziaceae (abstract id :564) publié dans Botany 2006, du 28 juillet 2006, disponible sous forme d'extrait sur internet http://www.2006.botanyconference.org/engine/search/index.php, est relatif à des pigments extraits de la plante *Ravenala madagascariensis* dont les structures chimiques n'ont pas encore été identifiées.

Cependant, le pigment a été identifié comme étant un complexe pigmentaire de protéines de couleur bleue.

Un deuxième article du même auteur Cary Pirone est publié dans Botany Interbiology 2007, du 7 juillet 2007, extrait disponible sur internet http://www.2007.botanyconference.org/engine/search/index.php.

Ici, il est indiqué que la recherche porte plus précisément sur l'identification de deux pigments inhabituels, un orange et l'autre jaune trouvés dans *P. Guayanense* et *S*. *Nicolai* dont la structure chimique a été identifiée par spectrométrie de masse et par RMN.

L'abrégé de la base de données Database WPI Week 200741 AN 2007-427 923 fait référence à la demande japonaise JP 2007 112 931 de Toyo Ink Manufacturing Co Ltd qui vise un pigment bleu obtenu par extraction avec un solvant organique alcoolique, une cétone aliphatique, etc. ajouté à un extrait de fleurs de Clitoria ternatea, qu'il est possible d'utiliser comme additif pour l'alimentation, des produits cosmétiques, pharmaceutiques et des encres.

Encore, l'article de Lianfu Zhang intitulé « Method for fast extracting lycopene », referencé dans la base de données Database WPI Week 2008, AN 2008-M13793 fait référence à une demande de brevet CN 101 121 631 de l'Université Jiangnan publiée le 13/2/08 pour l'extraction de lycopène.

Encore, l'abrégé publié dans la base de données Database WPI référence AN 2003-857 780, fait référence à une demande JP 2003/277 641 de Ichimaru Pharcos Inc relative à un nouveau dérivé pigmentaire extrait de racines de lithospermum ne procurant pas d'allergie de contact cutané ayant un domaine de stabilité de pH remarquablement amélioré, une bonne stabilité thermique et une capacité de filtration, utilisé comme colorant pour des compositions cosmétiques. Il est précisé que le dérivé de pigment de racine de lithospermum est un complexe formé par l'addition d'une solution contenant d'un ion de métal choisi parmi l'aluminium, le fer, le magnésium, le zinc, le cuivre et le manganèse, un composé d'acide bicarboxylique et un hydrolysat de protéine végétale (voir abrégé de ce document).

Encore, le document EP 1 191 071 est relatif à un agent colorant de type anthocyantine et une méthode de production de celui-ci à partir de matières organiques.

Enfin, le document WO 2008/129215 (Diana Naturals) est relatif à une composition alimentaire colorante comportant des colorants modifiés de la famille des anthocyanines ainsi qu'à un procédé de modification bathochrome de ces colorants.

On a recours également dans le domaine de la cosmétique à un deuxième type de pigments d'origine naturelle ou synthétique constitués de nacres. Ce deuxième type de pigments agit par un mécanisme différent pour conférer la coloration à la composition, puisque la production de couleurs est liée dans ce cas à un phénomène d'interférences ondulatoires de la lumière réfléchie sur la surface de la nacre.

Cet autre mode de production de couleurs n'est pas lié à un mécanisme d'absorption de la lumière par une substance chimique, mais à un phénomène d'interférences ondulatoires de la lumière réfléchie sur des structures présentes à la surface de l'objet observé.

Ces structures superficielles produisent ainsi un effet coloré, appelé iridescence, caractérisé par des couleurs qui changent selon l'angle d'observation ou selon l'angle d'incidence de la lumière d'éclairage.

Ces couleurs se distinguent des couleurs pigmentaires et sont souvent désignées sous le terme de « couleurs structurelles ».

Ce deuxième mode de production de couleur est présent chez de nombreuses espèces animales, en particulier parmi les insectes et les oiseaux.

On citera à titre d'exemple, certaines couleurs des plumes d'oiseaux ou des ailes de papillons.

Toutefois, alors que les couleurs structurelles sont assez fréquemment présentes dans le règne animal, elles sont très rares dans le monde végétal et n'ont été détectées que très récemment et chez un nombre limité d'espèces végétales.

La découverte de couleurs produites par interférence, chez quelques rares espèces végétales, a ainsi conduit les chercheurs à porter un nouveau regard sur les couleurs issues du monde végétal et à s'interroger sur l'avantage écologique pour la plante à développer de telles colorations iridescentes (Lee et al, Nature, 1975, 2445, 50-51).

Même si à ce jour le phénomène d'iridescence et les structures qui en sont responsables ne sont pas totalement élucidés, il a pu être établi que des ultrastructures généralement dénommées "iridisomes" sont responsables de l'effet coloré du produit résultant d'un phénomène d'interférence lumineuse à sa surface.

Parmi les espèces végétales dont certains tissus cellulaires comprennent des iridisomes, on peut citer les espèces végétales de la famille Strelitziaceae, en particulier l'espèce végétale Ravenala madagascariensis, des espèces végétales de la familles des Elaeocarpaceae, par exemple Elaeocarpus angustifolius Blume, des espèces végétales de la famille des Arraliaceae, en particulier l'espèce végétale Delarbrea michieana, des espèces végétales de la famille des Marattiaceae, en particulier l'espèce végétale Danaea nodosa, des espèces végétales de la famille Hymenophyllaceae, en particulier l'espèce végétale Trichomanes elegans, des espèces végétales parmi celles du genre Selaginella, par exemple Selaginella willdenowii, des espèces végétales de la famille des Athyriaceae, en particulier l'espèce végétale Diplazium tomentosum, des espèces végétales de la famille des Lindsaeaceae, en particulier l'espèce végétale Lindsaea lucida, des espèces végétales de la famille des Begoniaceae, en particulier l'espèce végétale Begonia pavonina, des espèces végétales de la famille des Melastolataceae, en particulier l'espèce végétale Phyllagathis rotundifolia.

Des chercheurs ont identifié et étudié de telles ultrastructures dans des cellules de l'épiderme du fruit de l'espèce végétale australienne Delarbrea micheana (Lee DW et al., Int. J. Plant Sci, 2000, 161 (2), 297-300).

Les auteurs ont émis l'hypothèse d'une coloration produite par un phénomène d'interférence dite « constructive ».

Ce phénomène d'interférence est provoqué par une région des cellules superficielles de l'épiderme de la plante, se présentant sous la forme d'une structure complexe et multicouche, à base de cellulose.

L'effet visuel produit par la plante est ainsi dépendant de l'épaisseur de ces multicouches responsables du mécanisme d'interférence lumineuse, épaisseur estimée à quelques dizaines de nanomètres.

Dans le cas de l'espèce Delarbrea micheana, ces ultrastructures sont localisées dans les cellules de l'épiderme du végétal, dans la région proche du milieu extérieur et ont une épaisseur estimée à environ 75 nm.

Bien qu'elles soient encore incomplètement caractérisées, il semble que ces ultrastructures végétales responsables de l'effet coloré produit par le végétal soient constituées de couches minces de cellulose hydratée et/ou d'agencements hélicoïdaux de fibrilles de cellulose, produisant dans ce cas un effet coloré homogène quel que soit l'angle d'observation du fait de leur répartition spatiale.

Les couleurs et nuances ainsi obtenues sont originales, de sorte que l'utilisation de telles ultrastructures végétales en tant que pigments structurels pourrait représenter une alternative aux procédés classiques de coloration utilisant des colorants ou des pigments sous la forme de molécules ou d'agrégats extractibles et purifiés.

Cependant, jusqu'à aujourd'hui, l'utilisation en tant que pigments colorés de ces ultrastructures végétales n'a jamais été envisagée, du fait que l'effet coloré provient d'un agencement fragile de macromolécules susceptible d'être dégradé lors de la mise en oeuvre du procédé d'extraction.

### BUTS DE L'INVENTION

Un but principal de la présente invention est de fournir une matière colorante à partir d'un matériel végétal coloré comprenant des ultrastructures végétales sans les dénaturer.

Un deuxième but principal de l'invention est de fournir une matière colorante à partir d'un matériel végétal coloré comprenant des ultrastructures végétales sans les dénaturer, de manière à les intégrer dans des compositions, en particulier dans des compositions cosmétiques, en vue de conférer à ces dernières une couleur qui est essentiellement la couleur de la partie colorée du végétal traité.

La présente demande décrit par ailleurs un procédé de production fiable, reproductible, et également peu coûteux de cette matière colorante à partir d'un matériel végétal coloré comprenant des ultrastructures végétales sans les dénaturer.

L'invention permet de résoudre pour la première fois ces problèmes techniques, de manière inattendue, sûre et fiable et utilisable à l'échelle industrielle et cosmétique.

### RESUME DE L'INVENTION

De façon tout à fait surprenante, les inventeurs de la présente invention sont parvenus à extraire de tissus végétaux comprenant ces ultrastructures végétales un produit coloré, ci-après désigné par "matière colorante de l'invention", dont la couleur très intense est celle de la partie colorée de la plante traitée.

On désigne par « matière colorante de l'invention », le produit coloré obtenu après extraction du tissu végétal dont on souhaite extraire la couleur. Ce produit coloré présente la couleur caractéristique du tissu végétal traité, couleur qui est liée à la présence des ultrastructures végétales ou "iridisomes", comme exposé précédemment.

Outre les possibilités d'identification de ces ultrastructures par des techniques telles que la microscopie électronique ou la spectrométrie de fluorescence X, l'homme du métier pourra s'assurer que ces ultrastructures responsables de la coloration ne sont pas dégradées par un suivi visuel.

Ainsi, la matière colorante de l'invention comprend les ultrastructures végétales directement responsables de l'effet coloré qui constituent la partie essentielle de cette matière colorante.

Une matière colorante décrite dans la présente demande peut en outre comprendre une fraction dite "fraction auxiliaire", ou encore « partie auxiliaire », extraite de la plante en même temps que les iridisomes, cette fraction pouvant être d'une nature variée selon la plante dont est extraite la matière colorante. Cette fraction auxiliaire qui ne participe pas directement à l'effet coloré, peut jouer un rôle de véhicule, de support, de stabilisant et ne nécessite pas d'être éliminée lors de la préparation d'une composition colorée. Il peut en effet s'avérer que la présence de cette fraction auxiliaire facilite la manipulation de la matière colorante de l'invention ou lui confère un effet complémentaire utile par exemple pour améliorer la texture de la composition dans laquelle la matière colorante est ajoutée pour la colorer.

Sans vouloir anticiper les résultats de la caractérisation structurelle des matières colorantes extraites, il semble bien à ce jour que la coloration du produit de l'invention soit liée à la non-dénaturation des ultrastructures végétales présentes dans les iridisomes qui se retrouvent par conséquent dans la matière colorante de l'invention.

Ainsi, les inventeurs de la présente invention ont pu extraire une matière colorante à partir d'un matériel végétal coloré comprenant des ultrastructures végétales sans les dénaturer, et à les intégrer dans des compositions, en vu de conférer à ces dernières une couleur qui est celle de la partie colorée du végétal traité.

Ainsi, l'extraction d'une matière colorante à partir d'un matériel végétal coloré comprenant des iridisomes, permet la mise en oeuvre d'une technique de coloration de compositions d'un type varié, telles que des compositions cosmétiques, des médicaments, des produits alimentaires, des encres, des teintures, des peintures et des produits applicables dans le domaine des arts graphiques et de la décoration en général.

Les matières colorantes ainsi obtenues sont susceptibles d'apporter de nouvelles nuances aux compositions dans lesquelles ils sont introduits.

Ces matières colorantes présentent d'autre part, une stabilité et une innocuité qui offrent une garantie supplémentaire de la préservation des qualités intrinsèques des compositions, notamment cosmétiques, dans lesquelles elles sont dispersées.

Elles sont enfin insolubles dans l'eau et dans tous les solvants habituellement utilisés, et compatibles avec les adjuvants non aqueux habituellement utilisés dans les compositions cosmétiques, ce qui rend leur utilisation particulièrement intéressante en tant que pigments colorés dispersés dans une composition cosmétique.

La présente demande décrit un extrait végétal coloré, ledit extrait étant obtenu à partir d'un matériel végétal formé par ou comprenant des tissus cellulaires colorés comprenant eux-mêmes des iridisomes.

Plus précisément, la présente demande décrit une nouvelle matière colorante qui doit ses qualités colorées et colorantes à un procédé permettant d'extraire la structure responsable de la coloration du matériel végétal, sans la dégrader, conduisant ainsi à un produit coloré et stable pouvant être utilisé comme pigment, notamment dans le domaine de la cosmétique.

La présente demande décrit également un procédé utilisé pour extraire cette matière colorante d'origine végétale sans la dénaturer.

La présente demande décrit également des compositions cosmétiques, notamment destinées au maquillage, renfermant de telles matières colorantes.

La présente demande décrit un procédé pour colorer une composition, en particulier une composition cosmétique, notamment destinée au maquillage.

L'invention a également pour objet un procédé de maquillage comprenant l'application d'une composition cosmétique renfermant une telle matière colorante.

### DESCRIPTION DETAILLEE DE L'INVENTION

Plus précisément, un premier objet de l'invention est une matière colorante obtenue à partir des arilles de l'espèce végétale Ravenala madagascariensis, ladite matière colorante renfermant des iridisomes responsables de la coloration et une matière cireuse,
ladite matière colorante étant obtenu par un procédé selon lequel on soumet les arilles à une étape de décompartimentation au moins partielle des cellules végétales des arilles renfermant lesdits iridisomes, en présence d'un milieu liquide,
ledit milieu liquide étant choisi dans le groupe constitué par l'eau ammoniaquée, l'acétone, l'acétate d'éthyle, les huiles essentielles végétales, le cyclohexane et l'heptane.

Un deuxième objet de l'invention concerne des compositions colorées cosmétiques, en particulier des compositions cosmétiques colorantes destinées au maquillage de la peau ou des phanères, contenant une dispersion de la matière colorante d'origine végétale du premier objet.

La présente demande décrit un procédé d'extraction d'une matière colorante d'origine végétale. Elle décrit également un procédé de préparation d'une composition colorée ou colorante, en particulier d'une composition cosmétique, comprenant l'introduction dans ladite composition d'au moins une matière colorante éventuellement obtenue par le procédé mentionné précédemment.

Un troisième objet de l'invention porte sur un procédé de maquillage de la peau ou des phanères tels que les cils, les cheveux ou les ongles, comprenant l'application sur au moins une partie de la peau ou des phanères de la composition cosmétique du deuxième objet.

Il est entendu que le deuxième objet de l'invention défini ci-dessus applicable aux compositions cosmétiques colorées ou colorantes, peuvent, sans difficulté pour l'homme du métier s'appliquer à des compositions de nombreux autres domaines, telles que les médicaments, les produits alimentaires, les encres, les teintures, les peintures et les produits applicables dans le domaine des arts graphiques et de la décoration en général.

D'autres caractéristiques et avantages de l'invention apparaîtront à la lecture de la description détaillée ci-après et des exemples qui suivent.

Comme cela a été exposé précédemment, l'invention résulte de la découverte par ses inventeurs qu'il était possible de préparer une matière colorante à partir d'une partie colorée d'une espèce végétale dont les tissus cellulaires renferment des iridisomes responsables de la coloration de cette partie de l'espèce végétale.

Comme exposé précédemment, les iridisomes responsables de la coloration de la plante dont on extrait la matière colorante de l'invention sont connus pour être constitués d'ultrastructures particulièrement fragiles. L'obtention de la matière colorante de l'invention nécessite donc la mise en oeuvre d'un procédé particulièrement doux dans lequel on respecte la structure de l'iridisome.

Il est apparu aux inventeurs de la présente invention qu'à cet effet, il était particulièrement avantageux de soumettre la partie de l'espèce végétale contenant les iridisomes responsables de la coloration à une étape au cours de laquelle on décompartimente au moins partiellement les cellules végétales comprises dans les tissus cellulaires renfermant les iridisomes, en présence d'un milieu liquide, de façon à libérer un produit coloré et à l'entraîner par l'intermédiaire de ce milieu liquide.

La production d'un effet coloré par ces iridisomes nécessite, semble-t-il, que ceux-ci présentent une structure après extraction qui soit non altérée par rapport à la structure des mêmes iridisomes dans les tissus cellulaires des végétaux ou parties de végétaux dans lesquels ils produisent l'effet coloré.

Les iridisomes présents dans le milieu liquide dans lequel ils sont recueillis, dès lors qu'ils ont conservé leur état non dénaturé et fonctionnel, produisent une coloration voisine ou identique à celle du tissu cellulaire à partir duquel ces structures sont extraites.

Pour décompartimenter au moins partiellement les cellules végétales, on utilise avantageusement au moins une source d'énergie externe.

Selon une variante particulièrement avantageuse de l'invention, la décompartimentation au moins partielle des cellules végétales est réalisée à l'aide d'au moins une source d'énergie externe, notamment par agitation mécanique, par exemple à l'aide d'un agitateur magnétique, d'un homogénéisateur ou d'un broyeur.

Selon une autre variante particulièrement avantageuse de l'invention, la décompartimentation au moins partielle des cellules végétales est réalisée sous l'effet des ultrasons, en présence d'un milieu liquide.

L'utilisation d'ultrasons s'est avérée particulièrement efficace dans le cadre de la présente invention.

En effet, sous l'effet des ultrasons dans un liquide, il se forme des micro-bulles, par l'effet bien connu de cavitation.

Ces micro-bulles sont pulsées par les ondes ultrasonores pour grandir jusqu'à une taille critique, après quoi elles implosent et restituent alors, lors de cet effondrement, leur énergie sous forme d'onde de choc.

L'homme du métier n'aura aucune difficulté à régler, dans des essais de routine la fréquence des ultrasons, notamment en contrôlant la couleur du liquide entraîné. Bien entendu, la fréquence optimale dépendra du matériel végétal traité.

Ainsi, dans le cas du matériel végétal utilisé selon l'invention, à savoir les arilles des graines de Ravenala madagascariensis, on choisira de préférence, des fréquences voisines de 27 kHz.

Pour décompartimenter plus efficacement les cellules végétales, il est également possible de soumettre le matériel végétal à une agitation mécanique combinée avec l'application d'ultrasons.

L'application des ultrasons ou de tout autre moyen de décompartimentation au moins partielle des cellules végétales a pour but de libérer les ultrastructures responsables de la coloration sans les dénaturer, de façon à libérer un produit coloré.

Le milieu liquide entraîne ainsi la structure libérée, en la séparant de la cellulose qui peut être éliminée ultérieurement, par exemple par filtration.

Ainsi, le milieu liquide est ensuite avantageusement soumis à une étape de filtration destinée à débarrasser le produit de l'invention d'au moins une partie des résidus à base de cellulose qui resteront sur le filtre.

D'une façon générale, la matière colorante selon l'invention est préparée selon un procédé permettant d'isoler les iridisomes du matériel végétal sans en dénaturer la structure qui est à l'origine de l'effet de coloration par interférence constructive.

Selon une variante optionnelle de ce procédé, on traite le matériel végétal préalablement à l'étape d'extraction elle-même, de façon à retirer une partie de la matière cireuse contenue dans ledit matériel végétal, par un procédé approprié. Ce pré-traitement du matériel végétal peut être avantageusement réalisé à l'aide de CO2 à l'état supercritique ou subcritique. La matière végétale partiellement « décirée » est ensuite soumise à l'étape d'extraction telle que décrite précédemment pour recueillir les iridisomes et la fraction cireuse restante. L'étape optionnelle de prétraitement permet d'obtenir une matière colorante dont les propriétés visuelles diffèrent sensiblement du produit obtenu sans prétraitement, en modifiant les proportions respectives d'iridisomes et de matière cireuse dans la matière colorante finalement obtenue.

Le procédé d'extraction des iridisomes responsables de l'effet coloré n'en dénature pas la structure.

Comme cela ressort de l'exposé qui suit, les iridisomes pourront être extraits en combinaison avec une fraction auxiliaire du matériel végétal.

La présence d'iridisomes dans des tissus colorés de végétaux peut être mise en évidence par différents moyens connus de l'homme du métier, notamment par microscopie électronique ou spectrométrie de fluorescence X.

Dans une mise en oeuvre préférée de l'invention, on soumet le tissu cellulaire coloré d'origine végétale comprenant lesdites structures, à des ultrasons en milieu liquide, le résultat de cette étape étant une décompartimentation des cellules végétales sous l'effet des micro-bulles formées par cavitation.

Sous l'effet des micro-bulles ainsi formées, les structures responsables de la coloration se détachent des cellules végétales dans lesquelles elles se trouvent stockées, sans être dénaturées.

Comme exposé précédemment, on connaît dans la littérature un certain nombre d'espèces végétales dont une partie des tissus contiennent des iridisomes. Toutes ces parties de plantes peuvent être traitées pour préparer une matière colorante décrite dans la présente demande. Sont ici décrites des espèces végétales de la famille des Elaeocarpaceae, par exemple Elaeocarpus angustifolius Blume, des espèces végétales de la famille des Arraliaceae, en particulier l'espèce végétale Delarbrea michieana, des espèces végétales de la famille des Marattiaceae, en particulier l'espèce végétale Danaea nodosa, des espèces végétales de la famille Hymenophyllaceae, en particulier l'espèce végétale Trichomanes elegans, des espèces végétales choisies parmi celles du genre Selaginella, par exemple Selaginella willdenowii, des espèces végétales de la famille des Athyriaceae, en particulier l'espèce végétale Diplazium tomentosum, des espèces végétales de la famille des Lindsaeaceae, en particulier l'espèce végétale Lindsaea lucida, des espèces végétales de la famille des Begoniaceae, en particulier l'espèce végétale Begonia pavonina, des espèces végétales de la famille des Melastolataceae, en particulier l'espèce végétale Phyllagathis rotundifolia. La partie de la plante choisie pour préparer une matière colorante dépend, bien entendu de l'espèce végétale retenue. Ainsi, le matériel végétal peut être une partie de plante ou la plante entière, mais peut en particulier être constitué d'une partie d'un fruit, d'une graine, ou d'une feuille ou d'un fruit entier, d'une graine entière, ou de la feuille entière à la condition que le matériel végétal comprenne ou soit formé d'un tissu cellulaire coloré comprenant des iridisomes. Le tissu cellulaire est préférentiellement formé par les couches cellulaires superficielles de la partie de la plante présentant un effet coloré par interférence, et en particulier par les couches cellulaires à la surface externe de la partie de plante, par exemple les cellules de l'épiderme du végétal. Le tissu peut être formé par exemple et, de façon non limitative, par l'épiderme du fruit, par l'enveloppe de la graine, ou le tissu de surface d'une feuille. Pour les plantes de forêts humides de Malaisie étudiées par Lee dans les publications citées précédemment, il s'agit des feuilles.

La matière colorante de l'invention est obtenue à partir des arilles de l'espèce végétale *Ravenala madagascariensis,* utilisées comme matériel végétal.

Le milieu liquide dans lequel l'extraction de la matière colorante de l'invention est réalisée est formé d'un solvant ou d'un mélange de solvants dans lequel les iridisomes sont insolubles mais qui permettra de les entraîner en combinaison avec la fraction cireuse du matériel végétal, elle-même séparée de la cellulose constitutive du tissu végétal sous l'effet des ultrasons.

L'homme du métier comprendra aisément que la composition du milieu liquide dans lequel s'opère la décompartimentation des cellules du tissu végétal est choisie en fonction de la nature du tissu végétal, ce choix étant fait pour entraîner un produit coloré associé à la matière cireuse du matériel végétal.

L'homme du métier comprendra également aisément que toute partie du matériel végétal ne participant pas à l'effet coloré et qui aurait été entraînée lors de l'extraction, ne doit pas nuire à l'effet coloré obtenu ou doit pouvoir être éliminé ultérieurement au cours d'au moins une étape de purification de la matière colorante de l'invention, avant son utilisation.

La partie finalement maintenue constitue ce que l'on désigne indifféremment par "partie auxiliaire" ou « fraction auxiliaire » au sens de l'invention, comme exposé précédemment. Les milieux liquides sont préférentiellement ceux dans lesquels la cellulose est insoluble et non dénaturée, et sont choisis parmi l'eau ammoniaquée, l'acétone, l'acétate d'éthyle, les huiles essentielles végétales, le cyclohexane et l'heptane.

La présente demande décrit en outre des milieux liquides choisis parmi ceux que l'homme du métier utilise pour mener un procédé d'extraction des couches minces, dans la mesure où ceux-ci n'attaquent pas le support en cellulose.

Le procédé d'obtention de la matière colorante de l'invention comprend également avantageusement une étape au cours de laquelle le milieu liquide récupéré à l'issue de l'étape de décompartimentation, est filtré de façon à éliminer les résidus végétaux et permettre de recueillir les structures responsables de l'effet coloré (iridisomes) éventuellement en présence de la fraction auxiliaire.

Après élimination des résidus végétaux, les structures végétales colorantes (essentiellement constituées d'iridisomes) sont recueillies et purifiées selon des procédures classiquement mises en oeuvre, pour former un extrait végétal apte à être utilisé en tant que pigment dans tout type de compositions pouvant en inclure.

Selon une première alternative, cette étape est réalisée par élimination directe du solvant ou du mélange de solvants formant le milieu liquide d'extraction.

Selon une deuxième mise en oeuvre possible, le milieu liquide constitué de la phase organique est refroidi et additionné d'eau froide. Les structures colorantes sont relarguées de la phase organique et floculent dans la phase aqueuse. Ces structures colorantes sont ensuite aisément recueillies par filtration.

On procède ensuite de façon optionnelle à une étape de purification de la matière colorante, par exemple en éliminant le ou les solvants formant le milieu liquide dans lequel elle a été extraite.

Comme exposé précédemment, il est intéressant d'entraîner au moyen du milieu liquide utilisé pour la mise en oeuvre du procédé d'extraction, non seulement la structure responsable de la coloration mais également la matière cireuse, dite fraction auxiliaire.

Ceci est en particulier le cas puisque les iridisomes se trouvent inclus dans une fraction cireuse de la partie colorée de l'espèce végétale.

On choisit, comme milieu liquide, un solvant ou un mélange de solvants approprié, de façon à extraire simultanément la structure responsable de la coloration, c'est-à-dire une matière colorante qui peut se présenter sous une forme cireuse, solide, ou pâteuse et le produit cireux, avec la possibilité d'utiliser directement ce mélange ou cette matière colorante dans une composition cosmétique formée ultérieurement.

Ainsi, comme cela ressort de la description détaillée qui suit, on met à profit, dans le cas de la matière colorante extraite des arilles des graines de l'espèce végétale Ravenala madagascariensis, qui constitue la matière colorante selon l'invention, le fait que les iridisomes sont contenus dans une partie cireuse de la plante.

Ravenala madagascariensis (encore appelé arbre du voyageur, ravenale Urania speciosa ou Urania madagascariensis) est une plante herbacée au stipe lacunaire, dont le tronc, à maturité, mesure environ dix mètres de hauteur, ce qui porte sa hauteur totale à environ 20 mètres.

Les fruits sont des capsules très dures à 6 loges contenant de nombreuses graines, entourées chacune d'une enveloppe, également appelée arille, dont la couleur bleue intense est assez rare chez les plantes.

Ces arilles contiennent près de 50% en poids d'une fraction cireuse encore mal connue.

Dans ce cas, on extrait en même temps, la structure responsable de la coloration bleue (iridisome) et la cire.

La description qui va suivre est donnée dans le cas des arilles des graines de l'espèce végétale Ravenala madagascariensis. Le procédé décrit est néanmoins directement adaptable à différents végétaux dans lesquels la partie contenant les iridisomes contient également une cire.

Selon une variante préférée de l'invention, la matière colorante extraite des arilles des graines de l'espèce Ravenala madagascariensis est un pigment bleu.

La matière colorante de l'invention est de préférence préparée selon un procédé qui permet d'extraire les iridisomes des tissus cellulaires de l'enveloppe de la graine sans dénaturer lesdits iridisomes. L'extrait ainsi obtenu est de couleur bleue.

Selon cette variante préférée du procédé de préparation de cette matière colorante, on soumet le matériel végétal formé par ou comprenant les arilles de l'espèce végétale Ravenala madagascariensis, à une agitation mécanique et/ou à l'action de micro-bulles formées par l'application d'ultrasons en milieu liquide.

Pour tous ces matériels végétaux présentant un caractère cireux, on met en oeuvre le procédé de décompartimentation, en particulier, sous l'effet des ultrasons, en présence d'un milieu liquide choisi pour entraîner la matière cireuse et les structures colorées (iridisomes), sous forme d'un produit coloré.

Selon une première variante avantageuse de ce procédé, le milieu liquide choisi est un solvant de la matière cireuse.

Un milieu particulièrement préféré est l'acétone.

Selon une variante avantageuse, ce procédé comprend une étape de précipitation du produit coloré, notamment par refroidissement du milieu liquide.

Selon cette variante, on ajoute de l'eau au milieu organique liquide, en particulier l'acétone, afin de faire relarguer la cire, et l'on refroidit. La cire flocule alors et entraîne le pigment coloré.

L'extrait cireux coloré est ensuite filtré, lavé et séché.

Une autre variante particulièrement intéressante du procédé permet d'éviter l'usage de solvant dont l'élimination complète est parfois délicate.

Selon cette variante, le milieu liquide est un milieu alcalin permettant de saponifier la matière cireuse, de façon à récupérer une solution basique colorée contenant la matière cireuse sous forme saponifiée ainsi que les structures colorées.

On utilise, dans ce cas de l'eau ammoniaquée pour réaliser l'étape de saponification.

On soumet ensuite de préférence le milieu liquide constitué de la solution basique colorée renfermant la matière colorante de l'invention à une étape de lavage par une solution acide, pour faire floculer la matière cireuse.

Ce deuxième type de procédé vise essentiellement à extraire une cire colorée renfermant la matière colorante de l'invention.

La cire, lors de sa saponification sous l'action de la solution basique permet, de faciliter l'extraction des ultrastructures.

Ainsi, l'obtention de la cire colorée se fait en deux temps selon ce procédé :
- un premier temps au cours duquel la solution basique extrait un "complexe" cire/composé coloré que l'on filtre et,
- un deuxième temps au cours duquel on acidifie le milieu basique de l'étape précédente par un acide organique, par exemple l'acide acétique, pour précipiter un produit « complexe » comprenant de la matière cireuse, insoluble en milieu acide, entraînant lors de sa précipitation les couches minces responsables du phénomène coloré, ce complexe est ensuite lavé, puis filtré et séché.

Dans le cas de l'espèce végétale Ravenala madagascariensis, les arilles riches en cire, sont traités de façon préférée selon un procédé permettant d'extraire les iridisomes en même temps que la cire : de préférence, les arilles sont traités par ultrasons dans l'eau ammoniaquée. De préférence on utilisera 1 ml d'ammoniaque à 20% (22° Bé) par litre d'eau et l'on maintient la température à environ 60°C durant 10mn. On peut réitérer une fois cette extraction. La cire est saponifiée et entraînée avec les iridisomes. Les liquides sont filtrés et neutralisés par l'acide acétique.

Lors de l'étape d'acidification susmentionnée, on adapte avantageusement le volume d'acide acétique de façon à ajuster le pH à une valeur voisine de 4, voire inférieure à 4, ce qui permet d'ajuster la taille des flocons de cire qui précipitent et en facilite la récupération.

La masse bleue cireuse qui a précipité est filtrée ; on la rince sur filtre et on la sèche à température modérée.

Selon une variante optionnelle de ce procédé, on traite les arilles de Ravenala madagascariensis à l'aide de CO2 à l'état supercritique ou subcritique, préalablement à l'extraction elle-même.

La matière colorante de l'invention peut être utilisée en tant que pigment dans des compositions colorées aptes à en inclure.

Ainsi, l'invention porte sur des compositions colorées, en particulier des compositions cosmétiques, contenant une dispersion de matière colorante telle que définie précédemment, agissant en tant que pigment.

Les compositions sont définies comme des compositions dans lesquelles l'agent colorant conserve ses propriétés colorantes sans que les autres composés n'en dénaturent la structure particulière.

Dans le domaine de la cosmétique, la forme de cette composition peut être par exemple celle d'un sérum, d'une lotion, d'une émulsion, telle qu'une crème de soin, d'un hydrogel, tel qu'un masque ou d'un mascara, d'un fond de teint, d'un fard à paupière ou d'un eyeliner, d'un stick, ou encore d'un patch.

Les compositions préférées sont celles comprenant des adjuvants gras, voire des adjuvants non aqueux ou essentiellement dépourvues d'eau.

Les compositions cosmétiques contenant la matière colorante de l'invention, comprennent avantageusement au moins un agent actif cosmétiquement acceptable et au moins un excipient cosmétiquement acceptable.

La composition cosmétique colorée peut être un produit de soin de la peau, et la composition cosmétique colorante sera un produit de maquillage de la peau ou des phanères.

Les compositions cosmétiques colorées qui comprennent l'agent colorant selon l'invention peuvent comprendre au moins un agent cosmétiquement actif choisi parmi les substances ayant une activité dépigmentante ou une activité éclaircissante de la peau; les substances ayant une activité amincissante ; les substances ayant une activité hydratante ; les substances ayant une activité calmante, apaisante ou relaxante ; les substances ayant une activité stimulant la microcirculation cutanée pour améliorer l'éclat du teint, en particulier du visage ; les substances ayant une activité sébo-régulatrice pour le soin des peaux grasses ; les substances destinées à nettoyer ou purifier la peau ; les substances ayant une activité anti-radicalaire ; les substances destinées à atténuer ou retarder les effets du vieillissement de la peau, en particulier la formation de rides, par une activité visant à favoriser le maintien de la structure de la peau et/ou à limiter la dégradation de la matrice extracellulaire des couches superficielles du derme et de l'épiderme et/ou à obtenir un effet protecteur, correcteur ou restructurant de la peau ; les substances ayant une activité anti-inflammatoire.

Outre la matière colorante selon l'invention, les compositions cosmétiques de l'invention comprennent au moins un excipient choisi parmi les pigments, les nacres, les colorants, les polymères, les agents tensioactifs, les agents de rhéologie, les parfums, les agents anti-oxydants et les conservateurs.

Les matières colorantes de l'invention sont particulièrement utiles en tant que pigments dans des compositions cosmétiques et notamment dans des compositions cosmétiques destinées au maquillage de la peau ou des phanères.

La présente demande décrit l'utilisation de la matière colorante de l'invention préparée à partir des arilles de graines de l'espèce végétale Ravenala madagascariensis, en tant qu'agent colorant, dans des compositions colorées ou colorantes aptes à en inclure, et plus particulièrement dans des compositions cosmétiques, en particulier des compositions destinées au maquillage de la peau ou des phanères tels que les cils.

On notera, que dans le cas des compositions cosmétiques, la présence de la cire peut constituer un avantage et que, dans ce cas, il n'y aura pas de nécessité de la séparer du reste du produit coloré. En particulier, dans le cas des arilles de Ravenala madagascariensis, la cire recueillie est très fine et peut s'avérer attrayante pour certaines compositions, notamment cosmétiques.

L'homme du métier comprendra aisément que la quantité de matière colorante contenue dans les compositions de l'invention dépend très largement du type de composition et de l'effet recherché.

D'une façon générale, et plus particulièrement dans le domaine de la cosmétique, on pourra utiliser la matière colorante de l'invention pour obtenir soit une composition colorée soit une composition colorante. L'homme du métier comprendra que les quantités de matière colorante dans ces deux types de composition, seront nécessairement différentes et dépendront bien entendu de la nature de cette matière colorante.

A titre d'exemple, plus précisément dans le domaine de la cosmétique, on pourra chercher à colorer une composition, par exemple une composition de soin cosmétique dont l'objet n'est pas de colorer la peau.

En particulier, dans le cas de l'utilisation de la matière colorante de couleur bleue intense extraite des arilles de la plante Ravenala madagascariensis, il sera possible pour l'homme du métier de choisir la quantité de cette matière colorante juste suffisante pour obtenir un effet azurant dans une composition de soin blanche, telle qu'une crème.

On pourra également colorer une composition cosmétique ayant pour fonction de colorer la peau ou les phanères, avec pour but d'obtenir une composition colorante, en particulier une composition destinée au maquillage.

Dans le cas particulier où la matière colorante est obtenue à partir des arilles des graines de l'espèce Ravenala masdagascariensis, on utilisera cette matière colorante de préférence dans des compositions comprenant une phase grasse et destinée à colorer la peau ou les phanères. Cette composition sera par exemple un mascara ou un rouge à lèvre.

L'invention a également pour objet un procédé de maquillage de la peau ou des phanères, en particulier des cils, des cheveux ou des ongles comprenant l'application sur au moins une partie de la peau ou des phanères d'une composition telle qu'elle a été décrite précédemment.

### EXEMPLE 1 : Préparation d'une matière colorante de l'invention, sous forme de cire colorée, à partir d'arilles de graines de Ravenala madagascariensis

On prépare une matière colorante selon de l'invention selon les étapes suivantes :
1- 100g d'arilles de graines de Ravenala madagascariensis sont placés dans la cuve d'un extracteur à ultrasons équipé de 4 dispositifs piézo-électriques (=400W), avec deux litres d'eau osmosée additionnée de 2ml d'ammoniaque à 20%- (22°Bé).
2- Afin de réaliser une décompartimentation des cellules végétales, on réalise une sonication (soumission du matériel végétal aux ultrasons). Celle-ci est réalisée à 55°C (température de départ) pendant 10 minutes environ, à une fréquence de 27 kHz. Durant cette opération, il ne se produit pas de changement sensible de la température (l'effet de cavitation des ultrasons engendre une montée en température qui compense les pertes dues au refroidissement spontané).
3- Après cette étape, on effectue une première filtration sur un tamis ou une toile de sérigraphie pour éliminer principalement les résidus cellulosiques. Le filtrat aqueux se présente sous l'aspect d'un lait bleu, contenant de la cire saponifiée.
4- On récupère des arilles retenus par le filtre et répète la sonication dans les mêmes conditions que celles précisées ci-dessus (temps, température, proportions d'ammoniaque, etc.).
5- Après filtration de la phase aqueuse de cette nouvelle opération de sonication, on réunit les jus filtrés.
6- La phase aqueuse ainsi obtenue est ensuite acidifiée à environ pH 4 par addition de 9 ml d'acide acétique à 75%. L'effet est immédiat: la couleur la cire flocule entraînant avec elle le pigment bleu, et se dépose au refroidissement.
7- Le processus est accéléré au réfrigérateur (température d'environ 4°C). L'eau surnageante est éliminée, et le précipité cireux bleu est lavé deux fois à l'eau osmosée pour éliminer les traces d'acétate d'ammonium qui s'est formé ainsi que l'acide résiduel.
8- Le précipité cireux bleu encore froid est ensuite récupéré par filtration sur un filtre en papier, de type papier joseph. Le filtrat est limpide
9- Le filtre retenant la cire bleue est séché à 25°C pour éliminer le plus d'eau possible.
Ce procédé permet d'obtenir environ 30g de cire colorée pour 100g d'arilles au départ.
Cette cire colorée constitue une matière colorante selon l'invention. Sa couleur est d'un bleu intense.
Elle pourra être utilisée comme pigment ou agent colorant pour colorer les compositions de l'invention.

### EXEMPLE 2 : Procédé de préparation d'une matière colorante de l'invention, sous forme de cire colorée, à partir d'arilles de graines de Ravenala madagascariensis

Suivant un procédé différent de celui décrit ci-dessus à l'exemple 1, les arilles de graines de Ravenala madagascariensis sont recouverts d'acétate d'éthyle ou d'acétone puis l'ensemble est placé dans une cuve d'extracteur à ultrasons du type de celle de l'exemple 1. Ils sont ensuite soumis à l'action des ultrasons pour réaliser la décompartimentation cellulaire, à la fréquence d'environ 27 kHz durant quelques minutes, jusqu'à épuisement de la matière végétale, puis l'extrait est filtré. La sonication dure environ 10 minutes. La cire libérée se dissout dans le solvant organique.

La phase organique ainsi obtenue est alors additionnée d'eau pour moitié de son volume. Cette addition d'eau provoque un relargage de la cire, qui dès lors flocule dans la phase aqueuse en entraînant avec elle le pigment bleu. On sépare alors la phase aqueuse contenant la cire colorée floculée par décantation.

Afin d'enrichir cette phase aqueuse en matière colorante bleue, on opère ensuite comme suit.

On réalise une extraction avec de nouveaux arilles comme précédemment décrit. La phase organique obtenue après sonication est filtrée, puis ajoutée à la phase aqueuse réservée précédemment. L'extrait cireux coloré contenu dans la phase aqueuse à l'état floculé est ainsi enrichi et sa couleur s'intensifie. On peut réitérer l'opération jusqu'à quatre fois avec toujours la même phase aqueuse.

A la fin de cette étape d'enrichissement, la cire colorée est séparée de la phase aqueuse par filtration sur papier extra fin, tel qu'un papier dénommé « papier Joseph » disponible dans le commerce.

On observera que l'on obtient ici un résidu cireux de couleur beaucoup plus intense que celui obtenu par le procédé de l'exemple précédent. Ce résidu est en effet plus concentré en pigment bleu, dans le mesure notamment où une partie significative de la cire est restée en solution dans le solvant, tandis que la quasi-totalité du pigment bleu a été entraînée dans la cire ayant floculé.

Le résidu cireux coloré ainsi obtenu est enfin débarrassé par évaporation des restes de solvant et d'eau par évaporation.

Ce résidu cireux de couleur très intense obtenu au terme du procédé décrit constitue une matière colorante selon l'invention.

### EXEMPLE 3 : Composition cosmétique anti-âge comprenant un pigment selon l'invention

La matière colorante obtenue selon l'exemple 1 est ajoutée à la phase huileuse d'une émulsion huile dans eau, pour la prévention du vieillissement de la peau dont la formule est décrite ci-dessous :

Les pourcentages sont exprimés en poids par rapport à la composition finale :
- Extrait végétal de Centella asiatica 0.1
- Colorant bleu selon l'exemple 1 2
- Tensio actif (Arlacel® 165 VP) 5
- Alcool cétylique 95% 1
- Alcool stéarylique 1
- Cire d'abeille 1.5
- Huile (Perleam®) 8.5
- Tri caprate/caprylate glycerides 3
- Huile silicone (diméthicone 100 CS) 1
- Polymère (Keltrol®) 0.35
- Soude 0.04
- EDTA tetrasodique poudre 0.1
- Conservateur 0,5
- Eau qsp 100

La composition est une crème anti-âge de couleur bleutée, dont l'activité vise à prévenir ou ralentir les signes du vieillissement cutané.

### EXEMPLE 4 : composition cosmétique pour le maquillage, comprenant une matière colorante selon l'invention

La matière colorante obtenue selon l'exemple 1 est ajoutée à la phase grasse d'une formule de mascara décrite ci-dessous :

| | |
|---|---|
| Colorant bleu selon l'exemple 1 | 10 |
| C18-36 triglycérides | 9,9 |
| Glycéryl stéarate | 12,0 |
| Autres colorant | 9,5 |
| Cire d'abeille | 4,6 |
| Cire de Carnauba | 2,2 |
| Triéthanolamine | 1,9 |
| SHELLAC | 1,9 |
| Acide stéarique | 1,9 |
| Acide palmitique | 1,9 |
| Glycéryl rosinate hydrogéné | 1,5 |
| PVP/VA copolymère | 0,95 |
| Lécithine | 0,95 |
| Conservateurs | 0,6 |
| Gomme xanthane | 0,4 |
| Phénoxyéthanol | 0,2 |
| Kératine hydrolysée | 0,15 |
| EDTA tétrasodique | 0,05 |
| Parfums | qs |
| Eau | qsp 100 |

Le mascara ainsi obtenu est de couleur bleu intense.

## Revendications

1. Matière colorante obtenu à partir des arilles de l'espèce végétale Ravenala madagascariensis, ladite matière colorante renfermant des iridisomes responsables de la coloration et une matière cireuse,
ladite matière colorante étant obtenu par un procédé selon lequel on soumet les arilles à une étape de décompartimentation au moins partielle des cellules végétales des arilles renfermant lesdits iridisomes, en présence d'un milieu liquide,
ledit milieu liquide étant choisi dans le groupe constitué par l'eau ammoniaquée, l'acétone, l'acétate d'éthyle, les huiles essentielles végétales, le cyclohexane et l'heptane.

2. Matière colorante selon la revendication 1, **caractérisée en ce que** la décompartimentation au moins partielle des cellules végétales est réalisée par agitation mécanique ou sous l'effet des ultrasons.

3. Matière colorante selon l'une des revendications 1 ou 2, **caractérisée en ce que** ledit procédé comprend en outre une étape de filtration destinée à débarrasser la matière colorante d'au moins une partie des résidus à base de cellulose.

4. Matière colorante selon la revendication 1, **caractérisée en ce que** ledit procédé comprend une étape de précipitation dudit colorant par refroidissement dudit milieu liquide.

5. Matière colorante selon la revendication 1, **caractérisée en ce que** le milieu liquide est une solution aqueuse d'alcali qui permet d'obtenir une solution basique colorée, et que le procédé comprend une étape de lavage par une solution acide de ladite solution basique colorée, pour faire floculer la matière cireuse.

6. Composition cosmétique, contenant un colorant selon l'une des revendications 1 à 5, et au moins un excipient choisi dans le groupe constitué par les pigments, les colorants, les nacres, les polymères, les agents tensioactifs, les agents de rhéologie, les parfums, les agents anti-oxydants et les conservateurs.

7. Composition selon la revendication 6, **caractérisée en ce qu'**il s'agit d'un produit de soin de la peau ou d'un produit de maquillage de la peau ou des phanères.

8. Composition selon la revendication 6, **caractérisée en ce qu'**il s'agit d'un sérum, d'une lotion, d'une émulsion, d'une crème de soin, d'un hydrogel, d'un masque, d'un mascara, d'un fond de teint, d'un fard à paupières, d'un eyeliner, d'un stick ou d'un patch.

9. Procédé de maquillage de la peau, des cils, des cheveux ou des ongles, **caractérisé en ce qu'**il comprend l'application - sur au moins une partie de la peau, des cils, des cheveux ou des ongles - d'une composition selon l'une des revendications 6 à 8.

## Patentansprüche

1. Farbmittel, das aus den Samenmänteln der Pflanzenart Ravenala madagascariensis erhalten wird, wobei der Farbstoff Iridosome, die für die Färbung verantwortlich sind, und ein wachsartiges Material enthält,
wobei das Farbmittel durch ein Verfahren erhalten wird, wobei die Samenmäntel einem Schritt des mindestens teilweisen Aufhebens der Kompartimentierung der Pflanzenzellen der Samenmäntel, die die Iridosome enthalten, in Anwesenheit eines flüssigen Mediums unterworfen werden,
wobei das flüssige Medium aus der Gruppe ausgewählt ist, die aus Ammoniakwasser, Aceton, Ethylacetat, pflanzlichen ätherischen Ölen, Cyclohexan und Heptan gebildet ist.

2. Farbmittel gemäß Anspruch 1, **dadurch gekennzeichnet, dass** das mindestens teilweise Aufheben der Kompartimentierung der Pflanzenzellen durch mechanisches Rühren oder unter der Wirkung von Ultraschall durchgeführt wird.

3. Farbmittel gemäß einem der Ansprüche 1 oder 2, **dadurch gekennzeichnet, dass** das Verfahren ferner einen Schritt des Filterns aufweist, der dazu bestimmt ist, den Farbstoff von mindestens einem Teil der Rückstände auf Zellulosebasis zu befreien.

4. Farbmittel gemäß Anspruch 1, **dadurch gekennzeichnet, dass** das Verfahren einen Schritt des Ausfällen des Farbstoffes durch Kühlen des flüssigen Mediums aufweist.

5. Farbmittel gemäß Anspruch 1, **dadurch gekennzeichnet, dass** das flüssige Medium eine wässrige Alkalilösung ist, die ermöglicht, eine gefärbte basische Lösung zu erhalten, und dass das Verfahren einen Schritt des Waschens der gefärbten basischen Lösung durch eine saure Lösung zum Flockulieren des wachsartigen Materials aufweist.

6. Kosmetische Zusammensetzung, umfassend einen Farbstoff gemäß einem der Ansprüche 1 bis 5 und mindestens einen Hilfsstoff, der aus der Gruppe ausgewählt ist, die aus Pigmenten, Farbstoffen, Perlmutt, Polymeren, Tensiden, Rheologiemitteln, Parfüms, Antioxidantien und Konservierungsmitteln gebildet ist.

7. Zusammensetzung gemäß Anspruch 6, **dadurch gekennzeichnet, dass** es sich um ein Hautpflegeprodukt oder um ein Produkt zum Schminken der Haut oder der Hautanhangsgebilde handelt.

8. Zusammensetzung gemäß Anspruch 6, **dadurch gekennzeichnet, dass** es sich um ein Serum, eine Lotion, eine Emulsion, eine Pflegecreme, ein Hydrogel, eine Maske, eine Mascara, eine Grundierung, einen Lidschatten, einen Eyeliner, einen Stift oder ein Patch handelt.

9. Verfahren zum Schminken der Haut, der Wimpern, der Haare oder der Nägel, **dadurch gekennzeichnet, dass** es das Auftragen - auf mindestens einem Teil der Haut, der Wimpern, der Haare oder der Nägel - einer Zusammensetzung gemäß einem der Ansprüche 6 bis 8 aufweist.

## Claims

1. A dyestuff that it is obtained from the arils of the *Ravenala madagascariensis* plant species, said dyestuff containing iridisomes that are responsible for the coloration, and a waxy material,
said dyestuff being obtained by means of a method according to which said arils are subjected to at least partial decompartmentalization of plant cells of arils containing iridisomes, in a liquid medium,
said liquid medium being selected from the group consisting of aqueous solutions of aqueous ammonia, acetone, ethyl acetate, plant essential oils, cyclohexane and heptane.

2. The dyestuff according to claim 1, **characterized in that** at least partial decompartmentalization of the plant cells is carried out by mechanical stirring or under the effect of ultrasound.

3. The dyestuff according to either claim 1 or 2, **characterized in that** said method also comprises a filtration step intended to free the dyestuff of at least a part of the cellulose-based residues.

4. The dyestuff according to claim 1, **characterized in that** said method comprises a step of precipitation of said colored product by cooling of said liquid medium.

5. The dyestuff according to claim 1, **characterized in that** said liquid medium is an alkaline aqueous medium which makes it possible to recover a colored basic solution, and **characterized in that** said process comprises a step of washing said colored basic solution with an acidic solution, so as to cause said waxy material to flocculate.

6. A cosmetic composition, **characterized in that** it contains a dyestuff as defined in one of claims 1 to 5, and at least one excipient selected in the group consisting of pigments, dyes, nacres, polymers, surfactants, rheology agents, fragrances, antioxidants and preservatives.

7. The composition according to claim 6, **characterized in that** it is a skin care product or a product for making up the skin or superficial body growths.

8. The composition according to claim 6, **characterized in that** it is a serum, a lotion, an emulsion, a care cream, a hydrogel, a mask, a mascara, a foundation, an eye shadow, an eyeliner, a stick or a patch.

9. A process for making up the skin, the eyelashes, the hair or the nails, **characterized in that** it comprises the application, to at least a part of the skin, the eyelashes, the hair or the nails, of a composition as defined in one of claims 6 to 8.
